(19) Europäisches Patentamt / European Patent Office / Office européen des brevets

(11) **EP 3 060 299 B1**

(12) **FASCICULE DE BREVET EUROPEEN**

(45) Date de publication et mention
de la délivrance du brevet:
**06.06.2018 Bulletin 2018/23**

(21) Numéro de dépôt: **14801946.6**

(22) Date de dépôt: **27.10.2014**

(51) Int Cl.:
**A61N 5/06** *(2006.01)*   **A61F 7/00** *(2006.01)*

(86) Numéro de dépôt international:
**PCT/EP2014/073031**

(87) Numéro de publication internationale:
**WO 2015/059312 (30.04.2015 Gazette 2015/17)**

(54) **DISPOSITIF D'IRRADIATION À INFRAROUGE**

INFRAROTSTRAHLUNGSVORRICHTUNG

INFRARED RADIATION DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **25.10.2013 FR 1360480**

(43) Date de publication de la demande:
**31.08.2016 Bulletin 2016/35**

(73) Titulaire: **Vital Tech
92100 Boulogne Billancourt (FR)**

(72) Inventeurs:
• **FAUCHON, Eric
F-92100 Boulogne Billancourt (FR)**
• **GAVSEVITCH, Alexandra
F-92100 Boulogne Billancourt (FR)**

(74) Mandataire: **Debay, Yves
Cabinet Debay
126, Elysee 2
78170 La Celle Saint Cloud (FR)**

(56) Documents cités:
WO-A1-2011/033329   CA-A1- 2 450 633
US-A- 6 004 344   US-A1- 2002 183 814

## Description

DOMAINE TECHNIQUE DE L'INVENTION

La présente invention se rapporte au domaine des traitements par radiation, plus particulièrement un dispositif d'irradiation par infrarouge.

ARRIÈRE-PLAN TECHNOLOGIQUE DE L'INVENTION

L'infrathérapie ou l'utilisation d'ondes infrarouges en thérapie permet une résonance du tissu musculaire augmentant les fonctions cellulaires et améliorant la circulation sanguine, le système cardiovasculaire ainsi que le système immunitaire. L'énergie radiante d'un dispositif d'irradiation à infrarouge fonctionne par fréquence de résonance sur la membrane cellulaire procurant une désintoxication en profondeur des couches supérieures du corps humain soumis à divers agents polluants. Par exemple, cette énergie radiante peut avoir un effet bénéfique sur le bien-être, sur la santé ou sur les performances sportives d'un utilisateur (9) d'un dispositif d'irradiation à infrarouge.

Le document US 2002/0183814 enseigne un dispositif d'irradiation à infrarouge comprenant un support destiné à recevoir un utilisateur allongé et des parties demi-cylindriques propres à recouvrir l'utilisateur allongé sur le support. Les parties demi-cylindriques comprennent des plaques chauffantes permettant une émission du rayonnement infrarouge dans le volume entre le support et les parties demi-cylindriques. Ce dispositif a le désavantage d'avoir un rayonnement infrarouge mal réparti qui peut être focalisé en un point ou un axe ou une zone limitée du corps de l'utilisateur qui surchauffe par rapport au reste du corps de l'utilisateur.

DESCRIPTION GÉNÉRALE DE L'INVENTION

La présente invention a pour objet d'obtenir un système d'irradiation par infrarouge améliorant les propriétés d'un rayonnement (6) infrarouge et en particulier possédant une répartition de l'émission infrarouge uniformisée.

A cet effet, l'invention concerne un dispositif d'irradiation à infrarouge comprenant un axe longitudinal parallèlement auquel sont disposés un support apte à recevoir un utilisateur et une partie recouvrante apte à recouvrir l'utilisateur, la partie recouvrante comprenant au moins une couche chauffante apte à émettre un rayonnement infrarouge dans au moins une partie du volume situé entre la face interne de la couche chauffante et le support, la ou les couches chauffantes comprenant au moins une plaque en carbone pour l'émission du rayonnement infrarouge, le dispositif étant caractérisé en ce que le profil de la couche chauffante, défini comme la section transversalement à l'axe longitudinal, suit une forme de courbe parabolique.

Selon une autre particularité, la courbe parabolique a pour équation $y = x^2 - x - 1$.

Selon une autre particularité, la partie recouvrante comprend en outre une couche réflectrice de rayonnement infrarouge.

Selon une autre particularité, la couche réflectrice suit le profil de la couche chauffante sur la face externe de la couche chauffante.

Selon une autre particularité, le support comprend une deuxième couche chauffante apte à émettre un rayonnement dans au moins une partie du volume entre la face interne de la première couche chauffante et le support.

Selon une autre particularité, une extrémité, selon l'axe longitudinal, de la partie recouvrante comprend une troisième couche chauffante fermant la partie recouvrante.

Selon une autre particularité, la partie recouvrante comprend au moins deux portions de profils paraboliques, la deuxième portion de la partie recouvrante étant apte à coulisser par rapport à la première portion parallèlement à l'axe longitudinal de sorte à s'escamoter dans ou sur la première portion (1a).

Selon une autre particularité, la première portion est rendue solidaire d'au moins une coulisse apte à guider dans un coulisseau.

Selon une autre particularité, la deuxième portion est rendue solidaire d'au moins un coulisseau sur sa face intérieure apte à coulisser sur au moins la coulisse de la première portion.

Selon une autre particularité, la partie recouvrante comprend une couche extérieure protégeant l'intérieur de la partie recouvrante.

Selon une autre particularité, la couche extérieure a une forme qui suit la forme de la couche chauffante.

Selon une autre particularité, la couche réflectrice comprend au moins une couche en aluminium.

Selon une autre particularité, la couche extérieure est en bois.

Selon une autre particularité, le support est en bois.

Selon une autre particularité, la surface interne de la partie recouvrante est recouverte d'un textile.

Selon une autre particularité, le dispositif d'irradiation comprend un système de relèvement de la partie recouvrante, la première portion ou la deuxième portion de la partie recouvrante étant fixée sur au moins le système de relèvement de la partie recouvrante, le système de relèvement de la partie recouvrante comprenant au moins un vérin de poussée dont une extrémité est fixée sur le support ou sur les pieds du support et l'autre extrémité est fixée sur la première ou la deuxième portion de la partie recouvrante de manière à ce que le vérin puisse exercer une poussée tendant à lever une extrémité de la partie recouvrante, l'autre extrémité de la partie recouvrante étant fixée sur le support par l'intermédiaire d'une articulation autorisant le fait que l'axe longitudinal de la partie recouvrante puisse faire un angle non nul avec le plan du support.

L'invention, avec ses caractéristiques et avantages, ressortira plus clairement à la lecture de la description faite en référence aux dessins annexés dans lesquels :

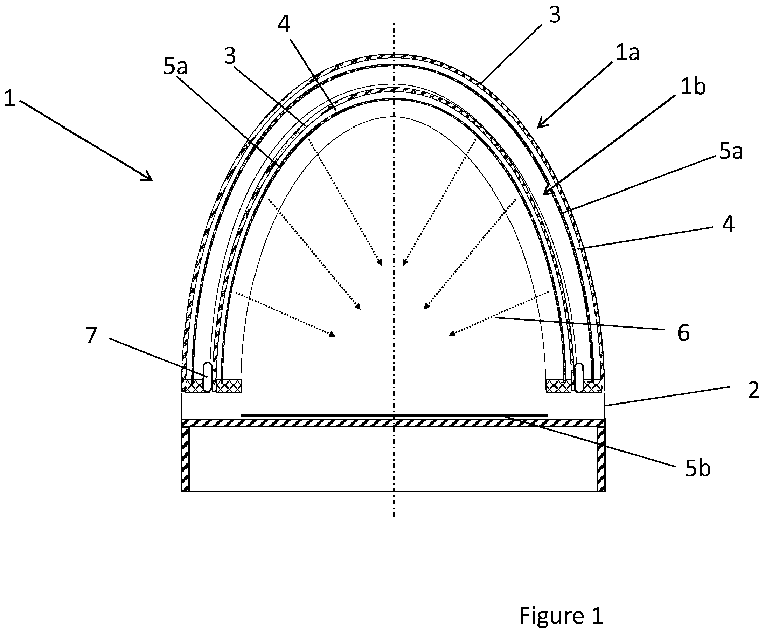

La figure 1 représente une coupe du dispositif selon un plan perpendiculaire à l'axe longitudinal du dispositif.

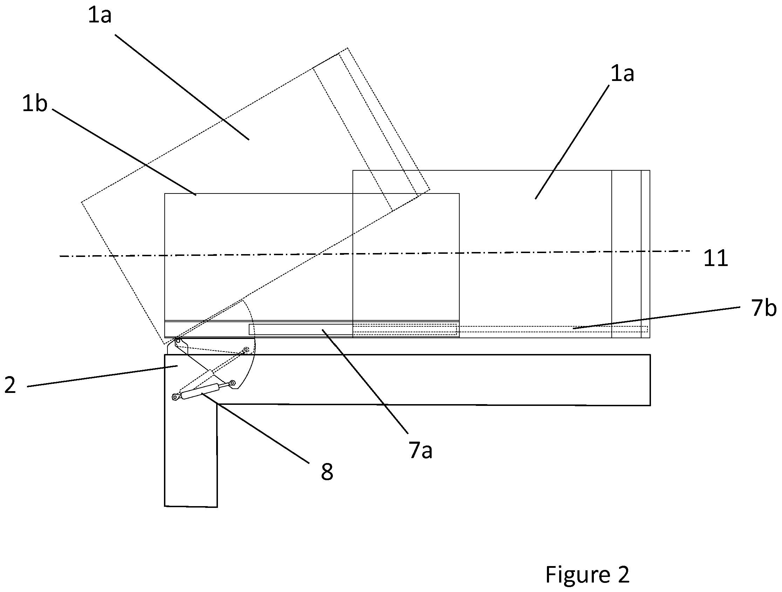

La figure 2 représente une vue de profil du dispositif selon deux positions de la partie recouvrante.

La figure 3 représente la répartition du rayonnement infrarouge dans un plan perpendiculaire à l'axe longitudinal du dispositif.

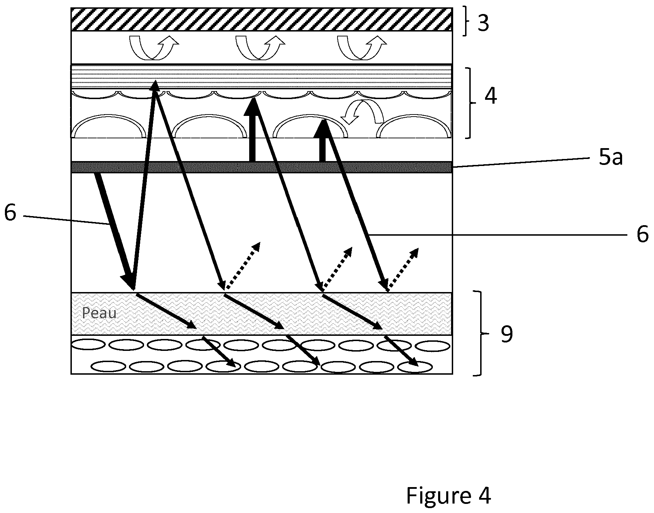

La figure 4 représente schématiquement le comportement des rayonnements infrarouge entre la partie recouvrante et l'utilisateur du dispositif.

La figure 5 représente une vue éclatée en perspective du dispositif.

DESCRIPTION DES MODES DE RÉALISATION PRÉFÉRÉS DE L'INVENTION

La suite de la description fera référence aux figures citées ci-dessus.

L'invention concerne un dispositif d'irradiation à infrarouge. Ce dispositif est parfois autrement appelé sauna individuel. Dans la suite de la description, le dispositif pourra être appelé dispositif d'irradiation ou simplement dispositif.

À la différence d'un sauna traditionnel qui réchauffe un corps indirectement en chauffant l'air ambiant, le dispositif d'irradiation à infrarouge permet de réchauffer le corps directement par la chaleur produite par le rayonnement (6) infrarouge. La température atteinte par le dispositif peut avantageusement se situer dans une plage allant de la température ambiante à environ 80°C, mais peut s'étaler jusqu'aux températures atteintes par les saunas traditionnels allant jusqu'à 105°C. La température ambiante est généralement une température égale à 20°C ou légèrement inférieure à 20°C, mais la température ambiante peut aller jusqu'à 35°C ou 40°C.

Le dispositif d'irradiation à infrarouge comprend un support (2).

Ce support (2) permet de recevoir un utilisateur (9), par exemple, allongé.

Le support (2) est surmonté de la partie (1) recouvrante qui permet de recouvrir et border les côtés de l'utilisateur (9) qui est, par exemple, allongé sur le support (2).

Le support (2) et la partie (1) recouvrante sont disposés selon un axe longitudinal du dispositif.

La partie (1) recouvrante comprend au moins une couche chauffante.

Dans certains modes de réalisation, cette couche comporte au moins une plaque en carbone. On désigne donc parfois, de façon non limitative, dans la présente description les couches chauffantes par le terme « plaques en carbone ». Les plaques en carbone sont produites généralement à partir d'un tissu polymère supraconducteur composé de carbone, de silice et de graphite. Le tissu est de préférence isolé entre deux feuilles de fibre de verre stratifié époxy et laminées à haute température. La forte densité et la répartition régulière du carbone permet une émission régulière du rayonnement (6) infrarouge sur toute sa surface. Une feuille en fibre de verre comprend généralement, sur deux bords opposés de la plaque en carbone, une matière conductrice d'électricité telle que le cuivre en contact avec le tissu et alimentée en électricité par l'intermédiaire d'un contrôleur (10). Par exemple, les deux bords ont deux bandes en cuivre alimentant le tissu.

La partie (1) recouvrante comprend au moins une couche (5a, 5b) chauffante.

Cette couche (5a, 5b) chauffante est apte à émettre un rayonnement (6) infrarouge dans au moins une partie du volume situé entre la face interne de la couche (5a, 5b) chauffante et le support (2).

Le rayonnement (6) infrarouge a comme particularité une émission d'ondes de la couche (5a, 5b, 5c) chauffante qui provoque une élévation de température de l'utilisateur (9) récepteur des ondes émises. De façon non limitative, les ondes émises ont des longueurs d'onde comprises entre 0,76 $\mu$m et 10 $\mu$m.

L'avantage de ce mode de chauffage réside dans le fait que les infrarouges ne sont pas ou peu absorbés par l'air, alors que les solides et les liquides les absorbent facilement.

Les longueurs d'onde sont inversement proportionnelles à la température de leur émetteur. D'après la loi de Wien, plus la température de l'émetteur est élevée, plus la longueur d'onde est courte.

L'absorption par les tissus biologiques des photons des ondes infrarouges modifie l'état de vibration ou de rotation moléculaire. En raison de leur faible énergie, les photons infrarouges ne peuvent pas produire d'ionisation, ni de réactions photochimiques. L'irradiation infrarouge est globalement perçue comme une source de chaleur procurant une détente et un bien-être, voire des avantages supplémentaires comme décrits dans la présente demande.

Dans certains modes de réalisation, les couches chauffantes du dispositif émettent un rayonnement (6) infrarouge long dans la plage de longueur d'onde comprise entre 6 $\mu$m à 19 $\mu$m.

La peau humaine est une matière complexe considérée comme un corps gris dont l'émissivité est proche de celle d'un corps noir. Elle dissipe sa chaleur en partie par radiation. Pour un corps noir, la loi de Stefan-Boltzmann intègre la notion d'émissivité qui représente un rapport entre l'énergie rayonnée par une surface subissant un flux incident et l'énergie que rayonnerait un corps noir à la même température. L'émissivité peut varier en fonction de la longueur d'onde. La loi de déplacement de Wien est obtenue par la dérivation de la loi de Planck. Elle donne la longueur d'onde correspondant au maximum de rayonnement spectral d'un corps noir en fonction de sa température. À partir de cette loi, on remarque que la peau à une température de 35°C émet un maximum d'énergie à une longueur d'onde de 9,4 $\mu$m. À une température de 37°C, la peau émet un maximum d'énergie à une longueur d'onde de 9,3 $\mu$m. À une température de 38,4°C, la peau émet un maximum d'énergie à une longueur d'onde de 9,3 $\mu$m.

Par conséquent, de préférence, les couches chauffantes du dispositif d'irradiation à infrarouge émettent à une longueur comprise entre 8,7 $\mu$m et 9,5 $\mu$m. Encore de préférence, elles émettent à une longueur d'onde de 9,3 $\mu$m.

De plus, il est connu que certains corps, notamment comprenant du carbone et du silicium peuvent émettre un rayonnement infrarouge qui se propage dans une direction privilégiée perpendiculaire à sa surface, par exemple comme cela est démontré dans la thèse de doctorat de Martine Laroche, (Rôle des ondes de surface dans la modification des propriétés radiatives de matériaux microstructurés. Application à la conception de sources infrarouges et à l'effet thermophotovoltaïque. École centrale, Paris, 2005). Ainsi, la ou les couches chauffantes, comprenant de préférence du carbone, du silicium et du graphite, utilisés dans certains modes de réalisation de la présente demande, émettent un rayonnement infrarouge dont la direction privilégiée est perpendiculaire à leur surface en tous points. Ce rayonnement directionnel peut être ressenti par le fait que la ou les couches chauffent l'espace face à elle et non pas à côté. Ceci a pour avantage de chauffer uniquement l'espace à l'intérieur du dispositif et de limiter le réchauffement ambiant dans la pièce où se trouve le dispositif. De plus, divers modes de réalisation tirent avantage de ce rayonnement directionnel en utilisant un profil particulier.

En effet, dans certains modes de réalisation préférés, la section de la couche (5a, 5b) chauffante transversalement à l'axe longitudinal suit avantageusement une forme représentative d'une courbe parabolique. On désigne ici cette forme de la section transversale par le terme profil et en particulier de profil parabolique. De plus, comme le dispositif peut comporter plusieurs couches chauffantes, on désigne ici cette couche chauffante à profil parabolique par le terme « première couche chauffante » mais ce terme ne doit pas être interprété de façon limitative car il désigne en fait la partie rayonnante du dôme au-dessus de l'utilisateur et composé d'une ou plusieurs couches chauffantes.

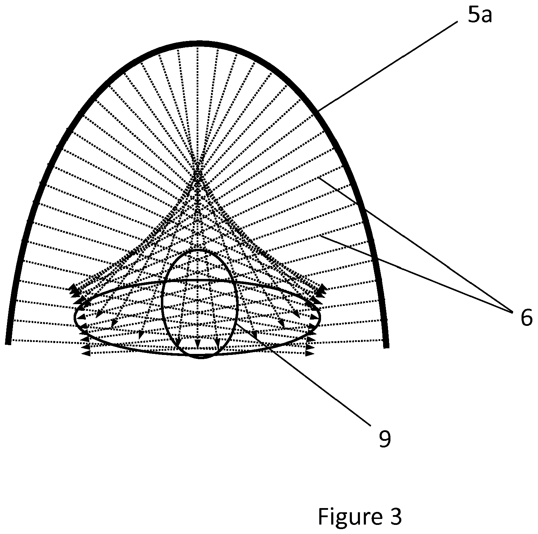

Le profil parabolique de la première couche (5a, 5b) chauffante permet d'obtenir une zone de convergence du rayonnement (6) infrarouge mieux répartie sur l'ensemble du corps de l'utilisateur (9). Par exemple comme représenté sur la figure 3, le rayonnement infrarouge est émis selon une direction privilégiée perpendiculaire à la surface de la couche chauffante en chaque point de la couche chauffante. La combinaison du profil parabolique avec cette direction privilégiée perpendiculaire permet une répartition du rayonnement de manière homogène. Dans la zone de convergence, la valeur de température générée en Kelvin peut être multipliée par un facteur de 1,4 pour deux rayons qui se croisent.

Si la première couche (5a, 5b) chauffante avait un profil de forme semi-circulaire, le rayonnement (6) infrarouge la zone de convergence se réduirait en un seul point qui serait le centre du cercle de la forme semi-circulaire. Ceci a pour conséquence une moins bonne répartition du rayonnement (6) infrarouge qui est focalisée sur un axe parallèle à l'axe longitudinal du dispositif. Tandis qu'avec un profil parabolique, la répartition peut avantageusement être ajustée pour suivre au moins approximativement la forme extérieure du corps de l'utilisateur présent à l'intérieur du dispositif. Ainsi, outre le confort de l'utilisateur, une telle répartition du chauffage permet au dispositif selon l'invention des applications en phlébologie telles que par exemple la réduction de varices, ce qui serait peu probable pour un dispositif de l'art antérieur.

Dans certains modes de réalisation, la courbe parabolique a pour fonction $y = x^2 - x - 1$. On notera que le nombre d'or $\dfrac{1+\sqrt{5}}{2}$ est la solution positive pour $x^2 - x - 1 = 0$, où x représente les coordonnées selon l'axe des abscisses parallèle au support (2) du dispositif et y représente les coordonnées selon l'axe des ordonnées perpendiculaire au support (2) du dispositif avec l'axe des ordonnées se dirigeant vers le sol.

Dans certains modes de réalisation, le dispositif comporte, par exemple au niveau du contrôleur (10), au moins un moyen d'affichage permettant d'afficher la longueur d'onde émise par la couche chauffante alimentée par l'intermédiaire du contrôleur (10). Un tel affichage peut être réalisé également sur un moyen d'affichage (13) du dispositif. En effet, dans certains modes de réalisation, le dispositif comprend un moyen d'affichage (13) permettant d'afficher des informations concernant le fonctionnement du dispositif telles que la température produit par les couches chauffantes, le temps d'utilisation du dispositif ou d'autres paramètres utiles pour le fonctionnement du dispositif.

Dans certains modes de réalisation, la partie (1) recouvrante du dispositif d'irradiation à infrarouge comprend aussi une couche (4) réflectrice de rayonnement (6) infrarouge sur la face externe de la couche chauffante. De préférence, le profil de la couche (4) réflectrice suit le profil de la couche (5a, 5b) chauffante. Cette couche (4) réflectrice permet de réfléchir le rayonnement (6) infrarouge qui n'a pas été absorbé par le corps de l'utilisateur (9) pour être renvoyé vers le corps de l'utilisateur (9). Ceci permet une économie d'énergie nécessaire pour émettre le rayonnement (6) infrarouge. Ceci permet également une sécurité du dispositif d'irradiation à infrarouge car la surface externe de la partie (1) recouvrante reste froide.

Dans une configuration, la couche (4) réflectrice comprend au moins un film ou une plaque en aluminium. Elle peut, en complément ou en alternative également comporter au moins un film ou une plaque contenant du cobalt, particulièrement efficace pour refléter les infrarouges.

Dans certaines configurations de la couche (4) réflectrice, elle comprend plusieurs composants. Dans une configuration préférée, les composants comprennent six films réflecteurs, deux couches en ouate et six couches en mousses.

Dans certains modes de réalisation, la partie (1) recouvrante du dispositif comprend une couche (3) extérieure protégeant l'intérieur de la partie (1) recouvrante. Dans certains modes de réalisation préférés, cette couche (3) est rigide. Dans certains modes de réalisation, cette couche (3) extérieure est en matière plastique ou en métal ou en bois. Dans certains modes de réalisation, la couche (3) extérieure peut être réalisée en fibres de verre ou en fibres de carbone ou en toute fibre moulable. Dans certains modes de réalisation, la couche (3) extérieure peut être réalisée par une combinaison des matériaux énumérés ci-avant.

La couche (4) réflectrice du dispositif est disposée entre la couche (3) extérieure et la couche chauffante. La couche chauffante est ainsi sans contact avec la couche (3) extérieure du dispositif, ce qui permet de limiter fortement les transferts d'énergie par conduction et procure une bonne efficacité thermique par rapport à l'art antérieur.

Dans certains modes de réalisation, la couche (3) extérieure a un profil de forme parabolique qui suit le profil de la couche chauffante. Par exemple, la couche (4) réflectrice, la couche (3) extérieure et la première couche (5a) chauffante sont fixes les unes par rapport aux autres. De préférence, elles sont fixées par des moyens de fixation supportant la chaleur et n'entraîne pas l'émission de vapeurs, voire permettant une isolation entre au moins 2 de ces 3 couches, par exemple grâce à un espace (e.g., une lame d'air) entre au moins 2 de ces couches, en particulier entre la couche (4) réflectrice et la couche extérieure (3). Les moyens de fixation sont par exemple des gorges dans lesquelles sont emboîtées les extrémités des différentes couches. Ces gorges sont, par exemple, disposés sur au moins deux bords situés aux extrémités opposées de la couche extérieure et dans lesquelles sont insérées les couches chauffante et réflectrice.

Dans certains modes de réalisation, le support (2) comprend une deuxième couche (non représentée) chauffante apte à émettre un rayonnement dans au moins une partie du volume entre la face interne de la couche chauffante et le support (2). Par exemple, la deuxième couche chauffante est intégrée dans un matelas, par exemple en matériau amortisseur ou confortable, posé sur le support (2).

Dans certains modes de réalisation, une première extrémité de la partie recouvrante selon l'axe longitudinal (11) est fermée. Dans certains de ces modes de réalisation, cette première extrémité de la partie (1) recouvrante comprend aussi une troisième couche (5c) chauffante qui chauffe la région des pieds de l'utilisateur.

Dans certains modes de réalisation, la deuxième extrémité est ouverte.

Dans certains modes de réalisation, la deuxième extrémité de la partie recouvrante comprend des moyens de fermeture amovible et/ou partielle de la deuxième extrémité. Dans certains de ces modes de réalisation, de tels moyens de fermeture amovible et/ou partielle sont avantageusement formés par un porte-serviette (12). Le porte-serviette est formé, par exemple, par une bande en textile élastique fixée sur la partie recouvrante de telle manière que la partie entre les deux extrémités de la bande peut être soulevée pour pouvoir introduire une serviette. Cette serviette permet alors de refermer la deuxième extrémité de la partie recouvrante.

Dans certains modes de réalisation, la surface interne de la partie recouvrante est recouverte d'un matériau évitant à l'utilisateur de pas être en contact direct avec la couche chauffante, comme par exemple un textile.

Dans certains modes de réalisation, la surface du support destinée à être en contact avec l'utilisateur est recouverte d'un matériau évitant à l'utilisateur de pas être en contact direct avec le support et l'éventuelle couche chauffante qu'il peut contenir. Un tel matériau peut être en textile et on peut lui associer un matériau amortisseur pour le confort du patient. Comme l'utilisateur transpire dans le dispositif, on préfère généralement que ce matériau soit imperméable ou qu'il disposé de manière amovible sur la surface.

Dans certains modes de réalisation, la partie (1) recouvrante comprend au moins deux portions dont une première portion (1a) et une deuxième portion (1b). La première portion (1a) peut s'escamoter sur ou dans la deuxième portion (1b) par coulissement de la première portion (1a) selon l'axe (11) longitudinal de la partie (1) recouvrante. Ainsi, par exemple, lorsque la première portion (1a) s'escamote sur la deuxième portion (1b), la première portion (1a) recouvre la deuxième portion (1b). On préfère en général que la portion qui recouvre l'autre soit celle qui correspond à l'extrémité près de laquelle l'utilisateur aura sa tête, en l'occurrence la première portion (1a) sur les figures illustratives et non limitatives.

Dans certains modes de réalisation, le coulissement est permis par un système (7) de coulissement. Selon une configuration particulière, un tel système de coulissement est obtenu par des glissières telles que des mécanismes de tiroir. Dans une autre configuration,, un tel système de coulissement peut comprendre au moins un coulisseau fixé sur la première portion (1a) de la partie (1) recouvrante. Le ou les coulisseaux sont fixés parallèle à l'axe (11) longitudinal de la partie (1) recouvrante à l'extrémité des branches de la forme parabolique. Le ou les coulisseaux sont aptes à se déplacer chacun dans une coulisse fixée sur le support (2) selon un axe (11) longitudinal de la partie (1) recouvrante. Dans une autre configuration, le coulisseau (7a) est fixé sur la surface extérieure de la deuxième portion (1b) parallèlement à l'axe (11) longitudinal de la partie (1) recouvrante au niveau des extrémités des branches de la forme parabolique. Les coulisses (7b) dans lesquelles les coulisseaux (7a) peuvent coulisser sont fixées sur la surface intérieure de la première portion (1a) parallèlement à l'axe (11) longitudinal de la partie (1) recouvrante au niveau des extrémités de la forme parabolique.

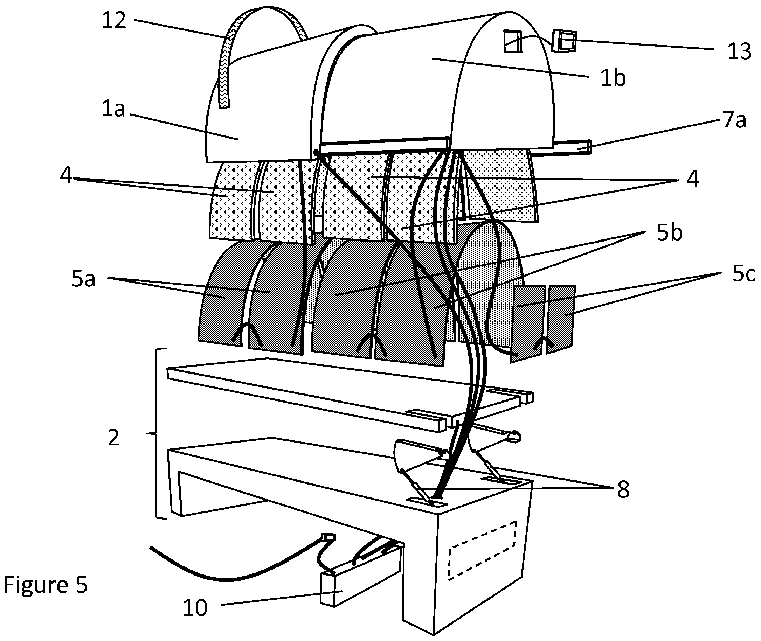

Dans la configuration illustrative et nullement limitative représentée sur la figure 5, la partie recouvrante (1) comprend une première portion et une deuxième portion. La première portion (1a) comprend une couche extérieure, deux couches (4) réflectrices et deux plaques en carbone (5a). La deuxième portion (1b) comprend également une couche extérieure, deux couches (4) réflectrices et deux plaques en carbone (5b). À une extrémité, la partie recouvrante est fermée par une plaque rigide sur laquelle sont fixées, par exemple deux plaques en carbone (5c). Le support (2) comprend également au moins une plaque en carbone.

Dans certains modes de réalisation, les plaques en carbone de la première portion sont alimentées indépendamment des autres plaques.

Dans certains modes de réalisation, les plaques de carbone et/ou les couches réflectrices sont fixées contre la couche extérieure par des moyens de fixation.

Dans certains modes de réalisation, les plaques en carbone de la deuxième portion sont alimentées indépendamment des autres plaques.

Dans certains modes de réalisation, les plaques en carbone du support (2) sont alimentées indépendamment des autres plaques.

Dans ces modes de réalisation où les plaques sont alimentées indépendamment les unes des autres, il est possible de moduler le rayonnement infrarouge selon les régions du volume compris entre la partie recouvrante et le support (2). Par exemple, l'alimentation des plaques en carbone de la première portion sont réglées de telle manière que le rayonnement infrarouge produit a une intensité moins importante que le rayonnement produit par les plaques en carbone de la deuxième portion.

Dans certains modes de réalisation, chaque portion et/ou le support comprend plusieurs plaques régulées indépendamment les unes des autres, de façon à fournir une modulation encore plus fine du rayonnement infrarouge, par exemple selon les zones à traiter dans le cas de la phlébologie.

Dans certains modes de réalisation, le dispositif comprend un moyen de réglage permettant de régler l'intensité de rayonnement des couches chauffantes.

Indépendamment de la forme du profil de la partie recouvrante, le dispositif d'irradiation comprend un système de relèvement de la partie recouvrante. La première portion (1a) ou la deuxième portion (1b) de la partie (1) recouvrante est fixée sur au moins le système de relèvement de la partie (1) recouvrante. Le système de relèvement de la partie (1) recouvrante comprend au moins un vérin (8) de poussée dont une extrémité est fixée sur le support (2) ou sur les pieds du support (2) et l'autre extrémité est fixée sur la première ou la deuxième portion (1b) de la partie (1) recouvrante de manière à ce que le vérin (8) puisse exercer une poussée tendant à lever une extrémité de la partie (1) recouvrante. L'autre extrémité de la partie (1) recouvrante est fixée sur le support (2) par l'intermédiaire d'une articulation autorisant le fait que l'axe (11) longitudinal de la partie (1) recouvrante puisse faire un angle non nul avec le plan du support (2). La force de poussée du ou des vérins (8) est déterminée en fonction de différentes position de la première portion (1a). Lorsque la première portion (1a) ne recouvre pas la deuxième portion (1b), la force de poussée du vérin (8) n'est pas suffisante pour soulever l'extrémité non fixée de la partie (1) recouvrante dont l'axe (11) longitudinal reste parallèle avec le plan du support (2). Lorsque la première portion (1a) recouvre la deuxième portion (1b), la force du vérin (8) est suffisante pour soulever l'extrémité fixée de la partie (1) recouvrante dont l'axe (11) longitudinal fait alors un angle non nul avec le plan du support (2). Un élément de butée permet de retenir la partie (1) recouvrante lorsqu'elle est soulevée par le ou les vérins (8) de limiter l'angle que fait l'axe (11) longitudinal de la partie (1) recouvrante et le plan du support (2).

Cet agencement présente l'avantage de faciliter l'ouverture du dispositif par l'utilisation lui-même ne poussant sur la première portion pour la faire coulisser et la première et la deuxième portion se soulèvent automatiquement sous l'action du vérin.

L'invention concerne donc éventuellement un dispositif comprenant un support (2) et une partie (1) recouvrante disposés selon un axe longitudinal du dispositif. La partie recouvrante comprend une première portion montée coulissante sur une deuxième portion fixée par un axe de pivotement sur le support. Le dispositif comprend un vérin monté entre la deuxième portion et le support (2) ou les pieds du support (2), la force de poussée du vérin étant adaptée pour que, d'une part, le poids des portions en position déployée empêche le soulèvement des deux portions, et, d'autre part, lorsque les portions sont escamotées, au moins partiellement, la force du vérin suffise à faire se relever automatiquement la partie recouvrante.

La présente description détaille différents modes de réalisation et configuration ou variantes en référence à des figures et/ou des caractéristiques techniques. L'homme du métier comprendra que les diverses caractéristiques techniques des divers modes ou configurations peuvent être combinées entre elles pour obtenir d'autres modes de réalisation et de configuration, à moins que l'inverse ne soit explicitement mentionné ou que ces caractéristiques techniques ne soient incompatibles. De même, une caractéristique technique d'un mode de réalisation ou d'une configuration peut être isolée des autres caractéristiques techniques de ce mode de réalisation à moins que l'inverse ne soit mentionné. Dans la présente description, de nombreux détails spécifiques sont fournis à titre illustratif et nullement limitatif, de façon à détailler précisément l'invention. L'homme de métier comprendra cependant que l'invention peut être réalisée en l'absence d'un ou plusieurs de ces détails spécifiques ou avec des variantes. À d'autres occasions, certains aspects ne sont pas détaillés de façon à éviter d'obscurcir et alourdir la présente description et l'homme de métier comprendra que des moyens divers et variés pourront être utilisés et que l'invention n'est pas limitée aux seuls exemples décrits.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Dispositif d'irradiation à infrarouge comprenant un axe (11) longitudinal parallèlement auquel sont disposés un support (2) apte à recevoir un utilisateur (9) et une partie (1) recouvrante apte à recouvrir l'utilisateur (9), la partie (1) recouvrante comprenant au moins une couche (5a) chauffante apte à émettre un rayonnement (6) infrarouge dans au moins une partie du volume situé entre la face interne de la couche (5a) chauffante et le support (2), la ou les couches chauffantes comprenant au moins une plaque en carbone pour l'émission du rayonnement infrarouge, le dispositif étant **caractérisé en ce que** le profil de la couche (5a) chauffante, défini comme la section transversalement à l'axe longitudinal, suit une forme de courbe parabolique.

2. Dispositif selon la revendication 1, **caractérisé en ce que** la courbe parabolique a pour équation $y = x^2 - x - 1$.

3. Dispositif selon la revendication 1, **caractérisé en ce que** la partie (1) recouvrante comprend en outre une couche (4) réflectrice de rayonnement (6) infrarouge.

4. Dispositif selon la revendication 3, **caractérisé en ce que** la couche (4) réflectrice suit le profil de la couche chauffante sur la face externe de la couche chauffante.

5. Dispositif selon au moins une des revendications 1 à 4, **caractérisé en ce que** le support (2) comprend une deuxième couche chauffante apte à émettre un rayonnement dans au moins une partie du volume entre la face interne de la première couche chauffante et le support (2).

6. Dispositif selon au moins une des revendications 1 à 5, **caractérisé en ce qu'**une extrémité, selon l'axe longitudinal, de la partie (1) recouvrante comprend une troisième couche (5c) chauffante fermant la partie (1) recouvrante.

7. Dispositif selon au moins une des revendications 1 à 6, **caractérisé en ce que** la partie (1) recouvrante comprend au moins deux portions de profils paraboliques, la deuxième portion (1b) de la partie (1) recouvrante étant apte à coulisser par rapport à la première portion (1a) parallèlement à l'axe (11) longitudinal de sorte à s'escamoter dans ou sur la première portion (1a).

8. Dispositif selon la revendication 7, **caractérisé en ce que** la première portion (1a) est rendue solidaire d'au moins une coulisse apte à guider un coulisseau.

9. Dispositif selon au moins une des revendications 7 et 8, **caractérisé en ce que** la deuxième portion (1b) est rendue solidaire d'au moins un coulisseau sur sa face intérieure apte à coulisser sur au moins la coulisse de la première portion (1a).

10. Dispositif selon au moins une des revendications 1 à 9, **caractérisé en ce que** la partie (1) recouvrante comprend une couche (3) extérieure protégeant l'intérieur de la partie (1) recouvrante.

11. Dispositif selon la revendication 10, **caractérisé en ce que** la couche (3) extérieure a une forme qui suit la forme de la couche chauffante.

12. Dispositif selon la revendication 3, **caractérisé en ce que** la couche (4) réflectrice comprend au moins une couche en aluminium.

13. Dispositif selon au moins une des revendications 10 et 11, **caractérisé en ce que** la couche (3) extérieure est en bois.

14. Dispositif selon au moins une des revendications 1 à 13, **caractérisé en ce que** le support (2) est en bois.

15. Dispositif selon au moins une des revendications 1 à 14, **caractérisé en ce que** la surface interne de la partie recouvrante est recouverte d'un textile.

16. Dispositif selon au moins une des revendications 1 à 15, **caractérisé en ce que** le dispositif d'irradiation comprend un système de relèvement de la partie recouvrante, la première portion (1a) ou la deuxième portion (1b) de la partie (1) recouvrante étant fixée sur au moins le système de relèvement de la partie (1) recouvrante, le système de relèvement de la partie (1) recouvrante comprenant au moins un vérin (8) de poussée dont une extrémité est fixée sur le support (2) ou sur les pieds du support (2) et l'autre extrémité est fixée sur la première ou la deuxième portion (1b) de la partie (1) recouvrante de manière à ce que le vérin (8) puisse exercer une poussée tendant à lever une extrémité de la partie (1) recouvrante, l'autre extrémité de la partie (1) recouvrante étant fixée sur le support (2) par l'intermédiaire d'une articulation autorisant le fait que l'axe (11) longitudinal de la partie (1) recouvrante puisse faire un angle non nul avec le plan du support (2).

**Patentansprüche**

1. Infrarotstrahlungsvorrichtung mit einer Längsachse (11), zu der parallel eine Auflage (2), die einen Benutzer (9) aufnehmen kann, und ein Abdeckteil (1) der den Benutzer (9) abdecken kann, angeordnet sind, wobei der Abdeckteil (1) wenigstens eine Heizschicht (5a) umfasst, die Infrarotstrahlung (6) in wenigstens einem Teil des Volumens aussenden kann, das sich zwischen der Innenfläche der Heizschicht (5a) und der Auflage (2) befindet, wobei die ein oder mehreren Heizschichten wenigstens eine Kohlenstoffplatte zum Aussenden der Infrarotstrahlung umfassen, wobei die Vorrichtung **dadurch gekennzeichnet ist, dass** das Profil der Heizschicht (5a), definiert als der Querschnitt transversal zur Längsachse, einer parabolischen Kurvenform folgt.

2. Vorrichtung nach Anspruch 1, **dadurch gekennzeichnet, dass** die parabolische Kurve der Gleichung $y = x^2 - x - 1$ entspricht.

3. Vorrichtung nach Anspruch 1, **dadurch gekennzeichnet, dass** der Abdeckteil (1) ferner eine Schicht (4) zum Reflektieren von Infrarotstrahlung (6) umfasst.

4. Vorrichtung nach Anspruch 3, **dadurch gekennzeichnet, dass** die reflektierende Schicht (4) dem Profil der Heizschicht auf der Außenfläche der Heizschicht folgt.

5. Vorrichtung nach einem der Ansprüche 1 bis 4, **dadurch gekennzeichnet, dass** die Auflage (2) eine zweite Heizschicht aufweist, die eine Strahlung in wenigstens einem Teil des Volumens zwischen der Innenfläche der ersten Heizschicht und der Auflage (2) aussendet.

6. Vorrichtung nach einem der Ansprüche 1 bis 5, **dadurch gekennzeichnet, dass** ein Ende, gemäß der Längsachse, des Abdeckteils (1) eine dritte Heizschicht (5c) umfasst, die den Abdeckteil (1) abschließt.

7. Vorrichtung nach einem der Ansprüche 1 bis 6, **dadurch gekennzeichnet, dass** der Abdeckteil (1) wenigstens zwei parabolische Profilabschnitte umfasst, wobei der zweite Abschnitt (1b) des Abdeckteils (1) in Bezug auf den ersten Abschnitt (1a) parallel zur Längsachse (11) gleiten kann, so dass er in oder auf den ersten Abschnitt (1a) eingezogen wird.

8. Vorrichtung nach Anspruch 7, **dadurch gekennzeichnet, dass** der erste Abschnitt (1a) fest mit wenigstens einer Gleitbahn verbunden ist, die einen Schieber führen kann.

9. Vorrichtung nach einem der Ansprüche 7 und 8, **dadurch gekennzeichnet, dass** der zweite Abschnitt (1b) fest mit wenigstens einem Schieber auf einer Innenfläche verbunden ist, der auf wenigstens der Gleitbahn des ersten Abschnitts (1a) gleiten kann.

10. Vorrichtung nach einem der Ansprüche 1 bis 9, **dadurch gekennzeichnet, dass** der Abdeckteil (1) eine Außenschicht (3) umfasst, die das Innere des Abdeckteils (1) schützt.

11. Vorrichtung nach Anspruch 10, **dadurch gekennzeichnet, dass** die Außenschicht (3) eine Form hat, die der Form der Heizschicht folgt.

12. Vorrichtung nach Anspruch 3, **dadurch gekennzeichnet, dass** die reflektierende Schicht (4) wenigstens eine Aluminiumschicht umfasst.

13. Vorrichtung nach einem der Ansprüche 10 und 11, **dadurch gekennzeichnet, dass** die Außenschicht (3) aus Holz ist.

14. Vorrichtung nach einem der Ansprüche 1 bis 13, **dadurch gekennzeichnet, dass** die Auflage (2) aus Holz ist.

15. Vorrichtung nach einem der Ansprüche 1 bis 14, **dadurch gekennzeichnet, dass** die Innenfläche des Abdeckteils mit einem Stoff bedeckt ist.

16. Vorrichtung nach einem der Ansprüche 1 bis 15, **dadurch gekennzeichnet, dass** die Strahlungsvorrichtung ein System zum Anheben des Abdeckteils umfasst, wobei der erste Abschnitt (1a) oder der zweite Abschnitt (1b) des Abdeckteils (1) an wenigstens dem Anhebsystem des Abdeckteils (1) befestigt ist, wobei das Anhebsystem des Abdeckteils (1) wenigstens einen Stoßzylinder (8) umfasst, von dem ein Ende an der Auflage (2) oder an den Füßen der Auflage (2) befestigt ist und das andere Ende am ersten oder zweiten Abschnitt (1b) des Abdeckteils (1) auf eine solche Weise befestigt ist, dass der Zylinder (8) eine Schubkraft ausüben kann, die ein Ende des Abdeckteils (1) anheben kann, wobei das andere Ende des Abdeckteils (1) an der Auflage (2) über ein Gelenk befestigt ist, das zulässt, dass die Längsachse (11) des Abdeckteils (1) einen Winkel von ungleich null mit der Ebene der Auflage (2) bilden kann.

## Claims

1. Infrared irradiation device comprising a longitudinal axis (11) parallel with which are disposed a support (2) capable of receiving a user (9) and a covering part (1) capable of covering the user (9), the covering part (1) comprising at least one heating layer (5a) capable of emitting infrared radiation (6) in at least one part of the volume situated between the internal face of the heating layer (5a) and the support (2), the heating layer or layers comprising at least one plate made of carbon for emitting the infrared radiation, the device being **characterised in that** the profile of the heating layer (5a), defined as the section transversely to the longitudinal axis, follows a form of parabolic curve.

2. Device according to claim 1, **characterised in that** the parabolic curve has the equation $y = x^2 - x - 1$.

3. Device according to claim 1, **characterised in that** the covering part (1) also comprises a reflective layer (4) which reflects infrared radiation (6).

4. Device according to claim 3, **characterised in that** the reflective layer (4) follows the profile of the heating layer on the external face of the heating layer.

5. Device according to at least one of claims 1 to 4, **characterised in that** the support (2) comprises a second heating layer capable of emitting radiation in at least one part of the volume between the internal face of the first heating layer and the support (2).

6. Device according to at least one of claims 1 to 5, **characterised in that** one end, in the longitudinal axis, of the covering part (1) comprises a third heating layer (5c) closing the covering part (1).

7. Device according to at least one of claims 1 to 6, **characterised in that** the covering part (1) comprises at least two portions of parabolic profiles, the second portion (1b) of the covering part (1) being capable of sliding in relation to the first portion (1a) parallel with the longitudinal axis (11) so as to be retracted into or over the first portion (1a).

8. Device according to claim 7, **characterised in that** the first portion (1a) is formed solidly in one piece with at least one slide channel capable of guiding a slider.

9. Device according to at least one of claims 7 and 8, **characterised in that** the second portion (1b) is formed solidly in one piece with at least one slider on its internal face capable of sliding on at least one slide channel of the first portion (1a).

10. Device according to at least one of claims 1 to 9, **characterised in that** the covering part (1) comprises an external layer (3) protecting the interior of the covering part (1).

11. Device according to claim 10, **characterised in that** the external layer (3) has a shape which follows to the shape of the heating layer.

12. Device according to claim 3, **characterised in that** the reflective layer (4) comprises at least one layer made of aluminium.

13. Device according to at least one of claims 10 and 11, **characterised in that** the external layer (3) is made of wood.

14. Device according to at least one of claims 1 to 13, **characterised in that** the support (2) is made of wood.

15. Device according to at least one of claims 1 to 14, **characterised in that** the internal surface of the covering part is covered with a textile.

16. Device according to at least one of claims 1 to 15, **characterised in that** the irradiation device comprises a system for lifting the covering part, the first portion (1a) or the second portion (1b) of the covering part (1) being fixed to at least the system for lifting the covering part (1), the system for lifting the covering part (1) comprising at least one thrust cylinder (8) one end of which is fixed to the support (2) or to the feet of the support (2) and the other end of which is fixed to the first or the second portion (1b) of the covering part (1) so that the cylinder (8) can exert a thrust tending to lift one end of the covering part (1), the other end of the covering part (1) being fixed to the support (2) through the intermediary of a hinge allowing the longitudinal axis (11) of the covering part (1) to form an angle of other than zero with the plane of the support (2).

Figure 1

EP 3 060 299 B1

Figure 2

Figure 3

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• US 20020183814 A

**Littérature non-brevet citée dans la description**

• **MARTINE LAROCHE.** Rôle des ondes de surface dans la modification des propriétés radiatives de matériaux microstructurés. Application à la conception de sources infrarouges et à l'effet thermophotovoltaïque. École centrale, 2005